# EUROPEAN PATENT APPLICATION

(11) **EP 4 467 131 A2**
(43) Date of publication of application: **27.11.2024**
(21) Application number: 24186625.0
(22) Date of filing: 17.07.2019
(51) Int. Cl.: A61K 9/48

(54) **MICROBIOTA RESTORATION THERAPY (MRT) COMPOSITIONS AND METHODS OF MANUFACTURE**

(30) Priority: 17.07.2018 US 201862699434 P
(62) Divisional of application: 19746410.0
(71) Applicant: Ferring B.V., 2132 JX Hoofddorp (NL)
(72) Inventor: FLUET, Gregory J., Minneapolis, Minnesota 55410 (US); BLOUNT, Kenneth F., Guilford, Connecticut 06437 (US)
(74) Representative: Marks & Clerk LLP

(57) **Abstract**

Microbiota restoration therapy compositions and methods for making and using microbiota restoration therapy compositions are disclosed. One example method is a method for enhancing the immune system of a patient. The method may include administering a microbiota restoration therapy composition to a patient with a primary condition. The method may also include administering a treatment for the primary condition to the patient. The treatment may be designed to treat the primary condition.

## Description

### Technical Field

The present disclosure pertains to compositions and methods for treating patients.

### Background

A method for enhancing the immune system of a patient is disclosed. The method comprises administering a microbiota restoration therapy composition to a patient with a primary condition; and administering a treatment for the primary condition to the patient.

### Brief Summary

Microbiota restoration therapy compositions and methods for making and using microbiota restoration therapy compositions are disclosed. Example methods include methods for treating cancer, methods for enhancing the immune system, or both. A method for enhancing the immune system of a patient is disclosed. The method comprises administering a microbiota restoration therapy composition to a patient with a primary condition; and administering a treatment for the primary condition to the patient.

Alternatively or additionally to any of the embodiments above, the primary condition includes cancer.

Alternatively or additionally to any of the embodiments above, the primary condition includes one or more of melanoma, non-squamous cell lung cancer, squamous cell lung cancer, renal cell carcinoma, head and neck tumors, bladder cancer, Hodgkin lymphoma, non-Hodgkin lymphoma, gastric cancer, colorectal cancer, multiple myeloma, esophageal cancer, breast cancer, glioblastoma, mediastinal B-cell lymphoma, other hematologic malignancies, testicular cancer, pancreatic cancer, lymphoma, cervical cancer, ovarian cancer, basal cell carcinoma, neuroblastoma, leukemia, and sarcoma.

Alternatively or additionally to any of the embodiments above, the treatment includes a chemotherapy drug.

Alternatively or additionally to any of the embodiments above, administering a treatment for the primary condition to the patient occurs after administering a microbiota restoration therapy composition to a patient with a primary condition.

Alternatively or additionally to any of the embodiments above, administering a microbiota restoration therapy composition to a patient with a primary condition enhances the immune system of the patient.

Alternatively or additionally to any of the embodiments above, administering a microbiota restoration therapy composition to a patient with a primary condition enhances the efficacy of the treatment.

Alternatively or additionally to any of the embodiments above, administering a microbiota restoration therapy composition to a patient with a primary condition includes administering the microbiota restoration therapy composition via an enema.

Alternatively or additionally to any of the embodiments above, administering a microbiota restoration therapy composition to a patient with a primary condition includes orally administering the microbiota restoration therapy composition.

A method for enhancing the immune system of a patient is disclosed. The method comprises: administering an immuno-oncology agent to a patient with a cancer to enhance the immune system of the patient, wherein the immuno-oncology agent includes a microbiota restoration therapy composition; and administering a cancer treatment to the patient.

Alternatively or additionally to any of the embodiments above, the cancer includes one or more of melanoma, non-squamous cell lung cancer, squamous cell lung cancer, renal cell carcinoma, head and neck tumors, bladder cancer, Hodgkin lymphoma, non-Hodgkin lymphoma, gastric cancer, colorectal cancer, multiple myeloma, esophageal cancer, breast cancer, glioblastoma, mediastinal B-cell lymphoma, other hematologic malignancies, testicular cancer, pancreatic cancer, lymphoma, cervical cancer, ovarian cancer, basal cell carcinoma, neuroblastoma, leukemia, and sarcoma.

Alternatively or additionally to any of the embodiments above, administering a cancer treatment to the patient occurs after administering an immuno-oncology agent to a patient with a cancer to enhance the immune system of the patient.

Alternatively or additionally to any of the embodiments above, administering an immuno-oncology agent to a patient with a cancer to enhance the immune system of the patient enhances the efficacy of the cancer treatment.

Alternatively or additionally to any of the embodiments above, administering a cancer treatment to the patient includes immune checkpoint therapy (ICT).

Alternatively or additionally to any of the embodiments above, administering a cancer treatment to the patient includes radiotherapy.

Alternatively or additionally to any of the embodiments above, administering a cancer treatment to the patient includes surgery.

A method for enhancing the immune system of a patient is disclosed. The method comprises: administering an immuno-oncology agent to a patient with a cancer to enhance the immune system of the patient, the immuno-oncology agent comprising a lyophilized fecal-derived microbiota and a capsule encapsulating the lyophilized fecal-derived microbiota; administering a cancer treatment to the patient.

Alternatively or additionally to any of the embodiments above, the primary condition includes one or more of melanoma, non-squamous cell lung cancer, squamous cell lung cancer, renal cell carcinoma, head and neck tumors, bladder cancer, Hodgkin lymphoma, non-Hodgkin lymphoma, gastric cancer, colorectal cancer, multiple myeloma, esophageal cancer, breast cancer, glioblastoma, mediastinal B-cell lymphoma, other hematologic malignancies, testicular cancer, pancreatic cancer, lymphoma, cervical cancer, ovarian cancer, basal cell carcinoma, neuroblastoma, leukemia, and sarcoma.

Alternatively or additionally to any of the embodiments above, administering a cancer treatment to the patient occurs after administering an immuno-oncology agent to a patient with a cancer to enhance the immune system of the patient.

Alternatively or additionally to any of the embodiments above, administering an immuno-oncology agent to a patient with a cancer to enhance the immune system of the patient enhances the efficacy of the cancer treatment.

A method for treating breast cancer is disclosed. The method comprises: administering an MRT composition to a patient with breast cancer prior to a primary breast cancer treatment; and treating the patient with the primary breast cancer treatment.

Alternatively or additionally to any of the embodiments above, wherein administering an MRT composition to a patient with breast cancer includes administering one or more oral capsules to the patient.

Alternatively or additionally to any of the embodiments above, administering one or more oral capsules to the patient includes administering four capsules or more per day to the patient.

Alternatively or additionally to any of the embodiments above, administering one or more oral capsules to the patient includes administering up to four capsules per day to the patient.

Alternatively or additionally to any of the embodiments above, administering one or more oral capsules to the patient includes administering four capsules or more per day to the patient for two weeks.

Alternatively or additionally to any of the embodiments above, administering one or more orals capsules to the patient includes administering four capsules or more per day to the patient for two or more weeks.

Alternatively or additionally to any of the embodiments above, administering one or more orals capsules to the patient includes administering four capsules or more per day to the patient for four weeks.

Alternatively or additionally to any of the embodiments above, each oral capsule comprises 1 × 10⁷ to 1 × 10¹⁰ colony forming units.

Alternatively or additionally to any of the embodiments above, each oral capsule comprises 1 × 10⁷ to 1 × 10¹⁰ colony forming units of viable bacteria.

Alternatively or additionally to any of the embodiments above, treating the patient with the primary breast cancer treatment includes surgery.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
FIG. 1 is a flowchart depicting an example process for manufacturing a microbiota restoration therapy (MRT) composition.
FIG. 2 is a flowchart depicting an example process for manufacturing an MRT composition.

While the disclosure is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the spirit and scope of the disclosure.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

"Mammal" as used herein refers to any member of the class Mammalia, including, without limitation, humans and nonhuman primates such as chimpanzees, and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be included within the scope of this term.

The term "cryopreservation," as used herein, refers to the process of cooling and storing biological cells, tissues, or organs at very low temperatures to maintain their viability. As a non-limiting example, cryopreservation can be the technology of cooling and storing cells at a temperature below the freezing point (e.g., 196 K) that permits high rates of survivability of the cells upon thawing.

The term "cryoprotectant," as used herein, refers to a substance that is used to protect biological cells or tissues from the effects of freezing.

As used herein, the term "microbiota" can refer to the human microbiome, the human microbiota or the human gut microbiota. The human microbiome (or human microbiota) is the aggregate of microorganisms that reside on the surface and in deep layers of skin, in the saliva and oral mucosa, in the conjunctiva, and in the gastrointestinal, genito-urinary, or vaginal tracts of humans. The human microbiome is comprised of bacteria but may also include fungi, phages, viruses, archaea, and the like. Some of these organisms perform tasks that are useful for the human host, but the function of the majority of the organisms that make up the human microbiome is unknown. Under normal circumstances, these microorganisms do not cause disease to the human host, but instead participate in maintaining health. Thus, this population of organisms is frequently referred to as "normal flora."

The population of microorganisms living in the human gastrointestinal tract is commonly referred to as "gut flora" or "gut microbiota." The microbial flora of the human gut encompasses a wide variety of microorganisms that may aid in digestion, the synthesis of vitamins, and creating enzymes (and/or other metabolites) including those not produced by the human body. These microbial flora and their metabolites can have direct and indirect impacts on the function of human immune system.

The phrase "microbiota restoration therapy composition" (MRT composition), as used herein, refers to compositions that may be designed to help restore the microbiota of a patient. Such compositions may be derived from human fecal material. In some instances, the MRT compositions may include human fecal material containing viable gut flora from a patient or donor, a diluent, and a cryoprotectant. Additional compositions include equivalent freeze-dried and reconstituted feces, or a "synthetic" fecal composition. The human fecal material may be screened for the presence of pathogenic microorganisms prior to its use. For example, the fecal material may be screened for the presence of *Clostridium* species including *C. difficile,* Norovirus, Adenovirus, enteric pathogens, antigens to *Giardia* species, *Cryptosporidia* species and other pathogens, including acid-fast bacteria, enterococci, including but not limited to vancomycin-resistant enterococci (VRE), methicillin-resistant *Staphylococcus aureus* (MRSA), extended spectrum beta-lactamase (ESBL), as well as any ova or parasitic bodies, or spore-forming parasites, including but not limited to Isospora, Clyslospora, and Cryptospora.

The process of fecal bacteriotherapy may include introducing an MRT composition (e.g., that may be a processed fecal drug-product derived from a fecal sample from a healthy donor or a donor having one or more desired characteristics) into a gastrointestinal tract of a patient to repopulate a healthy or desirable gut microbiota. The MRT composition may be introduced directly into the lower gastrointestinal tract (e.g., via enema or colonoscope), directly into the upper gastrointestinal tract (e.g., via endoscope, duodenoscope, gastro-nasal tube, etc.), orally (e.g., which may include an MRT composition encapsulated in a suitable capsule), or in another suitable manner. In at least some instances, the aim of the intervention is to restore the microbiota of the patient, treat a disease or condition, or otherwise improve the health of the patient.

The MRT compositions disclosed herein may be understood as including a population of bacteria and/or a bacterial suspension. In general, the population of bacteria/organisms may include and/or otherwise take the form of a full-spectrum drug product that may be manufactured by processing a human fecal sample (e.g., a fresh human fecal sample). The process for manufacturing the full-spectrum drug product may be similar to those processes disclosed in U.S. Patent No. 9,675,648, U.S. Patent Application Pub. No. US 2016/0361263, and/or U.S. Patent Application No. 16/009,157, the entire disclosures of which are herein incorporated by reference.

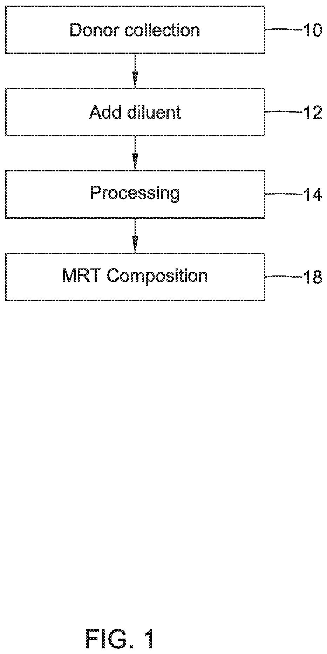

FIG. 1 is a flow chart summarizing one example process for producing an MRT composition. This process may form an MRT composition that is suitable for administration directly into the lower gastrointestinal tract (e.g., via enema or colonoscope) and/or directly into the upper gastrointestinal tract (e.g., via endoscope, duodenoscope, gastro-nasal tube, etc.). Generally, the process may include collecting a human fecal sample 10, adding a diluent to the fresh human fecal sample 12, and processing the sample 14 (e.g., which may include mixing the sample, filtering the sample, freezing, etc.) to form an MRT composition 16. In at least some instances, the diluent may include a cryoprotectant such as polyethylene glycol. For example, the diluent/cryoprotectant may include about 10-150 g/L or about 20-100 g/L, or about 30-90 g/L polyethylene glycol in saline. These are just examples. Other diluents, cryoprotectants, quantities/concentrations, etc. are contemplated.

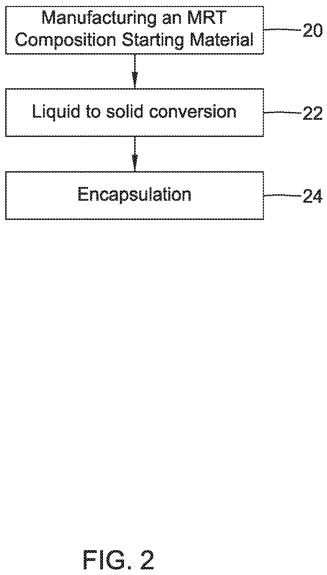

FIG. 2 is a flow chart summarizing another example process for manufacturing an MRT composition. This process may form an MRT composition that is suitable for oral administration. The process may include manufacturing/producing an MRT composition starting/precursor material 20. In at least some instances, the MRT composition starting material is the MRT composition manufactured according the process described with respect to FIG. 1. However, other processes/starting materials may be used. The MRT composition starting material may be processed and/or converted to a solid 24. This may include lyophilization or another suitable process. Once the sample has been converted/lyophilized, the sample may be further processed and the processed sample may be encapsulated 26, for example, into capsules suitable for oral delivery.

There is substantial research into the field of modulating various aspects of the immune system to treat cancer. This has resulted in advancements in cancer therapy through the development of novel drugs, including biologics, intended to modify how a patient's immune system reacts or fails to react to a malignancy. Examples of these therapies include but are not limited to CTLA-4 inhibitors, PD-1 inhibitors, PD-L1 inhibitors, and JAK1 inhibtors. They also can include bispecifc T-cell engaging (BiTE) antibodies or other drugs that are used to increase T-cell activation to target malignancies. Each of these drugs target various aspects of the patient's immune system in an effort to increase the patients' ability to attach the cancerous tumor(s) more aggressively than they were pre-treatment. The human microbiome and the various components of the human microbiota including bacteria, fungi, phages and their respective metabolites and proteins may affect the function of human organs and physiologic systems, including the immune system.

In some instances, the processes summarized in FIGS. 1-2 may be described or understood to be processes that include preserving and storing a highly diverse microbiota of a human donor that has a predefined characteristic (e.g., an MRT composition). In some instances, the MRT composition (e.g., that may include a preserved diverse microbiota) may be used to treat a cancer patient either as a mono-therapy or in conjunction with another treatment (e.g., chemotherapy, immuno-checkpoint therapy, immuno-oncology therapy, surgery, combination thereof, and/or the like) as a combination therapy.

The MRT compositions disclosed herein may be used to treat a number of different conditions. For example, the MRT compositions disclosed herein may be used to treat a number of disorders including *Clostridium difficile* infections (CDI) and/or other disorders including those disclosed herein. In some of these and in other instances, the MRT compositions disclosed herein may be used to help increase the functionality of human systems such as the immune system, help to treat diseases connected to the functioning of the immune system (auto-immune disease, ulcerative colitis (UC), psoriasis, etc.), enhance other therapies that may be associated with or otherwise rely on the functioning of the immune system (immuno-oncology treatments, t-cell inhibitors, cancer, etc.), and/or enhance other therapies. For example, an increase in the immune system functionality could increase the effectiveness of therapies that target diseases by using the human immune system as a weapon to attack these diseases. While not being wished to be bound by theory, the MRT compositions disclosed herein may help to increase immune system functionality by increasing T-cell activation, induction of interferon-gamma-producing CD8 T-cells, increasing the infiltration of T-cells into tumors, increasing infiltration of lymphocytes into tumors, increasing infiltration of interferon gamma-producing CD8 T-cells, decreasing CD4+ regulatory T-cells in tumors, spamming with other cells (CD3, PD1, FoxP3 producing T-cells, etc.), and/or combinations thereof. These are just examples.

In some instances, a clinician planning to treat a patient with a primary condition may treat the patient with an MRT composition, for example in conjunction with and/or prior to treating the primary condition. When doing so, rather than directly treating the primary condition, the MRT composition may be aimed at enhancing the patient's immune system, enhancing the effectiveness of treatment of the primary condition, etc. For example, a physician planning to treat a patient (e.g., a patient with cancer) with an immuno-oncology drug could first treat the patient with MRT composition to improve the capabilities of the patient's immune system to respond to the immuno-oncology and, accordingly, enhance the effectiveness of the immuno-oncology drug. In some of these and other instances, a physician planning to treat a patient (e.g., a patient with cancer) with radiotherapy could similarly first treat the patient with MRT composition to improve the capabilities of the patient's immune system to respond to the radiotherapy and increase the effectiveness of this therapy. In some of these and other instances, a physician planning to treat a surgically treat patient (e.g., a patient with cancer) could similarly first treat the patient with MRT composition to improve the capabilities of the patient's immune system to respond to the surgery, increase the effectiveness of the surgery, and/or otherwise improve long-term outcomes. In these instances, the MRT composition may be considered to be a neoadjuvant therapy (e.g., and/or a neoadjuvant therapy composition).

In some of these and in other instances, the MRT compositions disclosed herein may help reduce the side effects of cancer treatments. For example, some cancer treatments (e.g., including immuno-oncology treatments, radiotherapy, chemotherapy, etc.) may impact the digestive system of the patient and/or have other side effects. The side effects may include immuno-therapy-induced colitis. Administering an MRT composition in conjunction with, after and/or prior to a cancer treatment may help to reduce these and other side effects.

The MRT compositions disclosed herein may be used in conjunction with a number of different immune system related therapies including the use of immune checkpoint inhibitors, bispecific T-cell activating antibody therapies (e.g., which may be able to bind two antigens simultaneously and may be able to act as a bridging agent for two different cell types), adoptive T-cell transfer therapy, etc. For example, treatment methods are contemplated that includes administration of an MRT composition in conjunction with (e.g., before, during, or after) a cancer treatment that includes immune checkpoint therapy. Immune checkpoint therapy may include the use of agents such as PD-1 inhibitors, PD-L1 inhibitors, CTLA-4 agents, and the like, and/or combination thereof. Other therapies that may be improved by the administration of the MRT compositions disclosed herein may include adoptive T-cell therapies, T cell-activating bispecific antibodies (TABs), intratumoral injection of unmethylated CG dinucleotides (e.g., CpG therapies), cell therapies (e.g., CAR-T therapies/drugs), combinations thereof, and/or the like.

In some instances, administering an MRT composition to a patient may change the number of T cells or regulatory T cells. In other instances, administering an MRT composition to a patient may modulate the metabolic function of regulatory T cells, for example in cases where administering an MRT composition to a patient reduces the adverse effects of colitis. Thus, administering an MRT composition to a patient can ramp up the immune system, which increases the effectiveness against the tumors, while at the same time modulate the immune system so that the adverse effects of a systemic increase in immune system activity does not lead to or otherwise cause colitis This dichotomy or dual effect on the immune system is part of the uniqueness of treating patients with an MRT composition, which differentiates treatment with an MRT composition from other therapies and/or adjunctive therapies like surgery, chemotherapy, and/or radiation with ICTs (e.g., would only increase the immune system, rather than increase and modulate the immune system in a manner similar to treatment with an MRT composition).

Methods are contemplated that include administering an MRT composition to a patient in order to improve, enhance, or supplement the patient's immune system. These methods may include methods for treating auto-immune diseases/disorders, UC, psoriasis, cancer, and/or other conditions. Some example cancers that may be responsive to immuno-oncology treatments in combination with MRT compositions disclosed herein may include melanoma, non-squamous cell lung cancer, squamous cell lung cancer, renal cell carcinoma, head and neck tumors, bladder cancer, Hodgkin lymphoma, non-Hodgkin lymphoma, gastric cancer, colorectal cancer, multiple myeloma, esophageal cancer, breast cancer, glioblastoma, mediastinal B-cell lymphoma, other hematologic malignancies, testicular cancer, pancreatic cancer, lymphoma, cervical cancer, ovarian cancer, basal cell carcinoma, neuroblastoma, leukemia, sarcoma, and the like, and/or combinations thereof.

In some instances, methods are disclosed for treating conditions and/or for enhancing the immune system of a patient. The methods may include administering a microbiota restoration therapy composition to a patient with a primary condition. In some instances, the primary condition may include a cancer such as those disclosed herein. Thus, in some instances, the microbiota restoration therapy composition may be considered to be an immuno-oncology agent or composition. As described herein, the microbiota restoration therapy composition may be manufacturing using a suitable process such as those disclosed herein.

In addition to what is disclosed herein, the present disclosure is directed to compositions, methods of manufacture and methods of treatment utilizing MRT composition for the treatment of a number of different conditions. For example, the present disclosure pertains to compositions, methods of manufacture and methods of treatment utilizing MRT composition for the treatment of *Clostridium difficile* infections (CDI). CDI is a common nosocomial infection and is frequently associated with severe morbidity and mortality, especially in elderly patients. While CDI treatment is one example use for the MRT compositions disclosed herein, this is not intended to be limiting. Other diseases and/or conditions are contemplated. Some of the medical conditions that may be desirably impacted by treatment with MRT compositions may include cardiovascular and/or peripheral vascular disease, allergies, obesity, hypoglycemia, constipation, celiac sprue (e.g., celiac disease), cancer including gastrointestinal cancer (e.g. gastrointestinal cancer is at least one of stomach cancer, esophageal cancer, colon cancer gallbladder cancer, liver cancer, pancreatic cancer, colorectal cancer, anal cancer, melanoma, lung cancer, bladder cancer, prostate cancer, and gastrointestinal stromal tumors), hepatic encephalopathy, myoclonus dystonia, sacrolileitis, spondyloarthropathy, spondylarthritis, proximal myotonic myopathy; an autoimmune disease nephritis syndrome, autism, travelers' diarrhea, small intestinal bacterial overgrowth, chronic pancreatitis, a pancreatic insufficiency, chronic fatigue syndrome, benign myalgic encephalomyelitis, chronic fatigue immune dysfunction syndrome, Parkinson's Disease (PD), amyotrophic lateral sclerosis (ALS), multiple sclerosis (MS), degenerative neurological diseases, Grand mal seizures or petit mal seizures, Steinert's disease, chronic infectious mononucleosis, epidemic myalgic encephalomyelitis, idiopathic thrombocytopenic purpura (ITP), an acute or chronic allergic reaction obesity, anorexia, irritable bowel syndrome (IBS or spastic colon) Crohn's disease, irritable bowel disease (IBD), colitis, ulcerative colitis or Crohn's colitis, chronic infectious mononucleosis, epidemic myalgic encephalomyelitis, acute or chronic urticarial, lupus, rheumatoid arthritis (RA) or juvenile idiopathic arthritis (JIA), pre-diabetic syndrome, fibromyalgia (FM), Type I or Type II diabetes, asthma, acute or chronic insomnia, migraines, urinary tract infections (UTIs), and attention deficit/hyperactivity disorder (ADHD).

In the case of humans, the present disclosure encompasses methods of treatment of chronic disorders associated with the presence of abnormal enteric microflora. Such disorders include but are not limited to those conditions in the following categories: gastrointestinal disorders including irritable bowel syndrome or spastic colon, functional bowel disease (FBD), including constipation predominant FBD, pain predominant FBD, upper abdominal FBD, non ulcer dyspepsia (NUD), gastroesophageal reflux, inflammatory bowel disease including Crohn's disease, ulcerative colitis, indeterminate colitis, collagenous colitis, microscopic colitis, chronic Clostridium difficile infection, pseudomembranous colitis, mucous colitis, antibiotic associated colitis, idiopathic or simple constipation, diverticular disease, AIDS enteropathy, small bowel bacterial overgrowth, celiac/coeliac disease, polyposis coil, colonic polyps, chronic idiopathic pseudo obstructive syndrome; chronic gut infections with specific pathogens including bacteria, viruses, fungi and protozoa; viral gastrointestinal disorders, including viral gastroenteritis, Norwalk viral gastroenteritis, rotavirus gastroenteritis, AIDS related gastroenteritis; liver disorders such as primary biliary cirrhosis, primary sclerosing cholangitis, fatty liver or cryptogenic cirrhosis; rheumatic disorders such as rheumatoid arthritis, non-rheumatoid arthritis, non-rheumatoid factor positive arthritis, ankylosing spondylitis, Lyme disease, and Reiter's syndrome; immune mediated disorders such as glomerulonephritis, haemolytic uraemic syndrome, juvenile diabetes mellitus, mixed cryoglobulinemia, polyarthritis, familial Mediterranean fever, amyloidosis, scleroderma, systemic lupus erythematosus, and behcets syndrome; autoimmune disorders including systemic lupus, idiopathic thrombocytopenic purpura, Sjogren's syndrome, haemolytic uremic syndrome or scleroderma: neurological syndromes such as chronic fatigue syndrome, migraine, multiple sclerosis, amyotrophic lateral sclerosis, myasthenia gravis, Guillain-Barre syndrome, Parkinson's disease, Alzheimer's disease, Chronic Inflammatory Demyelinating Polyneuropathy, and other degenerative disorders; psychiatric disorders including chronic depression, schizophrenia, psychotic disorders, manic depressive illness; regressive disorders including, Aspergers syndrome, Rett syndrome, attention deficit hyperactivity disorder (ADHD), and attention deficit disorder (ADD); the regressive disorder, autism; sudden infant death syndrome (SIDS), anorexia nervosa; dermatological conditions such as chronic urticaria, acne, dermatitis herpetiformis and vasculitis disorders; and cardiovascular and/or vascular disorders and diseases.

Globally, the increase in the prevalence of drug resistant organisms has created many challenges for clinicians that may pose public health risks. Infections by drug resistant organisms (e.g., vancomycin-resistant *Enterococcus* (VRE)) and *Clostridium difficile* infection share similar risk factors. VRE is a nosocomial pathogen that can be a complication among transplant and immune compromised patients. VRE carriers may also be at increased risk for infection due to VRE and also be a potential source of VRE transmissions to others. VRE shedding in stool increases with antimicrobial exposures and decreases with normalization of the intestinal microbiota after antimicrobials are discontinued. Accordingly, normalization of intestinal microbiota may not only be useful for treating *Clostridium difficile* infections (including chronic infections), these treatments may also be useful for treating infections by drug resistant organisms (e.g., VRE and/or other drug resistant organisms including those disclosed herein).

In some instances, the microbiota restoration therapy compositions (and/or fecal bacteriotherapy compositions) disclosed herein may be used to treat patients with infections by drug resistant organisms and/or multi-drug resistant organisms (MDRO). The drug resistant organisms may be resistant to antimicrobial agents (e.g., antibiotics, antivirals, antifungals, antiparasitics, other drugs, combinations thereof, and the like) and may include drug resistant micro-organisms such as bacteria, viruses, fungi, parasites, etc. The infections that can be treated by the microbiota restoration therapy compositions disclosed herein may be along the digestive tract or along other systems of the patient.

The MRT compositions disclosed herein may be used to treat infections by a variety of drug resistant organisms such as vancomycin-resistant enterococci (VRE), methicillin-resistant *Staphylococcus aureus* (MRSA), extended-spectrum β-lactamase producing gram-negative bacteria, *Klebsiella pneumoniae* carbapenemase producing gram-negative bacteria, multi-drug resistant gram negative rods bacteria (e.g., such as *Enterobacter* species, *E.coli, Klebsiella pneumoniae, Acinetobacter baumannii,* and *Pseudomonas aeruginosa*), drug resistant Enterobacter species, multi-drug resistant tuberculosis (e.g., *Mycobacterium tuberculosis*), drug resistant *staphylococci,* drug resistant *enterococci,* drug resistant *gonococci,* drug resistant *streptococci* (e.g., including *Streptococcus pneumoniae*), drug resistant *salmonella,* drug resistant gram negative bacteria, drug resistant *Candida,* drug resistant HIV, drug resistant influenza virus, drug resistant cytomegalovirus, drug resistant herpes simplex virus, drug resistant malaria, drug resistant *Plasmodium vivax,* drug resistant *Plasmodium falciparum,* drug resistant *Toxoplasma gondii,* and the like, and/or other drug resistant organisms. These are just examples.

Treatment of infections by drug resistant organisms with the microbiota restoration therapy compositions disclosed herein may include treating patients with no prior history of infection by or caused by a drug resistant organism, treating patients with a single prior infection by a drug resistant organism, treating patients with two or more (e.g., two, three, four, five, six, or more) prior infections by a drug resistant organism, etc. In some instances, the microbiota restoration therapy compositions may be used to treat a patient with three prior infections by a drug resistant organism. In other instances, the microbiota restoration therapy compositions may be used to treat a patient with two prior infections by a drug resistant organism if the prior infections resulted in hospitalization, if the prior or current infections require treatment with toxic drugs, or if the prior infections were all from the same organism.

In some instances, MRT compositions can be administered to a patient using an enema or other suitable technique. However, it may be desirable to orally administer an MRT composition. In order to prepare an MRT composition in a form suitable for oral administration, a number of steps may be carried out. Generally, these steps may include collecting a fecal sample, processing the fecal sample, lyophilizing or "freeze-drying" the processed fecal sample (or otherwise converting the processed fecal sample from a liquid to a solid), adding one or more additives and/or excipients, and forming an oral form of the MRT composition from the lyophilized material and additives (e.g., a tablet, capsule, liquid preparation, or the like). Some additional details regarding at least some of these steps are disclosed herein.

### Examples

The disclosure may be further clarified by reference to the following Examples, which are prophetic in nature and serve to exemplify some embodiments, and not to limit the disclosure in any way.

### Example 1: Treatment of breast cancer

Treatment with an oral MRT composition prior to surgical breast cancer treatment may significantly increase cytotoxic T cells and/or reduce regulatory T cells in both peripheral blood and tumors. The increase in cytotoxic T cells and/or reduction of regulatory T cells may enhance the efficacy of the cancer treatment. In some of these and in other instances, treatment with an MRT composition may modulate the metabolic function of regulatory T cells.

The oral MRT composition may be the same or similar to any of the oral MRT compositions disclosed in U.S. Patent Application No. 16/009,157 (published as U.S. Patent Application Pub. No. US 2018/0289750) and/or U.S. Patent 10,226,431, the entire disclosures of which are herein incorporated by reference.

Patients with breast cancer will be grouped into cohorts based on their breast cancer profile. Cohort 1 will include patients that are estrogen receptor (ER) and/or progesterone receptor (PR) positive only (e.g. no human epidermal growth factor receptor 2 (HER2) amplification or overexpression). Cohort 2 will include patients that test positive for human epidermal growth factor receptor 2 (HER2) along with ER and/or PR. Cohort 3 will include patients that are negative for ER, PR, and HER2.

Patients from each cohort will be administered the oral MRT composition according to a dosing regimen. Dosing regimen one will include administering 4 capsules of the oral MRT composition per day for 2 weeks (e.g., 2 weeks prior to surgery). Each capsule may contain about 1 × 10⁷ to 1 x 10¹⁰ colony forming units (e.g., of viable bacteria). Dosing regimen two will include administering 4 capsules of the oral MRT composition per day for 4 weeks (e.g., 4 weeks prior to surgery). Treatment with the oral MRT composition may enhance the efficacy of the cancer treatment (e.g., surgical treatment of breast cancer).

It should be understood that this disclosure is, in many respects, only illustrative. Changes may be made in details, particularly in matters of shape, size, and arrangement of steps without exceeding the scope of the disclosure. The invention's scope is, of course, defined in the language in which the appended claims are expressed.

The present invention will now be described by reference to the following numbered clauses:
Clause 1. A method for enhancing the immune system of a patient, the method comprising: administering a microbiota restoration therapy composition to a patient with a primary condition; and administering a treatment for the primary condition to the patient.
Clause 2. The method of clause 1, wherein the primary condition includes cancer.
Clause 3. The method of any one of clauses 1-2, wherein the primary condition includes one or more of melanoma, non-squamous cell lung cancer, squamous cell lung cancer, renal cell carcinoma, head and neck tumors, bladder cancer, Hodgkin lymphoma, non-Hodgkin lymphoma, gastric cancer, colorectal cancer, multiple myeloma, esophageal cancer, breast cancer, glioblastoma, mediastinal B-cell lymphoma, other hematologic malignancies, testicular cancer, pancreatic cancer, lymphoma, cervical cancer, ovarian cancer, basal cell carcinoma, neuroblastoma, leukemia, and sarcoma.
Clause 4. The method of any one of clauses 1-3, wherein the treatment includes a chemotherapy drug.
Clause 5. The method of any one of clauses 1-4, wherein administering a treatment for the primary condition to the patient occurs after administering a microbiota restoration therapy composition to a patient with a primary condition.
Clause 6. The method of any one of clauses 1-5, wherein administering a microbiota restoration therapy composition to a patient with a primary condition enhances the immune system of the patient.
Clause 7. The method of any one of clauses 1-6, wherein administering a microbiota restoration therapy composition to a patient with a primary condition enhances the efficacy of the treatment.
Clause 8. The method of any one of clauses 1-7, wherein administering a microbiota restoration therapy composition to a patient with a primary condition includes administering the microbiota restoration therapy composition via an enema.
Clause 9. The method of any one of clauses 1-7, wherein administering a microbiota restoration therapy composition to a patient with a primary condition includes orally administering the microbiota restoration therapy composition.
Clause 10. A method for enhancing the immune system of a patient, the method comprising: administering an immuno-oncology agent to a patient with a cancer to enhance the immune system of the patient, wherein the immuno-oncology agent includes a microbiota restoration therapy composition; and administering a cancer treatment to the patient.
Clause 11. The method of clause 10, wherein the cancer includes one or more of melanoma, non-squamous cell lung cancer, squamous cell lung cancer, renal cell carcinoma, head and neck tumors, bladder cancer, Hodgkin lymphoma, non-Hodgkin lymphoma, gastric cancer, colorectal cancer, multiple myeloma, esophageal cancer, breast cancer, glioblastoma, mediastinal B-cell lymphoma, other hematologic malignancies, testicular cancer, pancreatic cancer, lymphoma, cervical cancer, ovarian cancer, basal cell carcinoma, neuroblastoma, leukemia, and sarcoma.
Clause 12. The method of any one of clauses 10-11, wherein administering a cancer treatment to the patient occurs after administering an immuno-oncology agent to a patient with a cancer to enhance the immune system of the patient.
Clause 13. The method of any one of clauses 10-12, wherein administering an immuno-oncology agent to a patient with a cancer to enhance the immune system of the patient enhances the efficacy of the cancer treatment.
Clause 14. The method of any one of clauses 10-13, wherein administering a cancer treatment to the patient includes immune-checkpoint therapy.
Clause 15. The method of any one of clauses 10-14, wherein administering a cancer treatment to the patient includes radiotherapy.
Clause 16. The method of any one of clauses 10-15, wherein administering a cancer treatment to the patient includes surgery.
Clause 17. A method for enhancing the immune system of a patient, the method comprising: administering an immuno-oncology agent to a patient with a cancer to enhance the immune system of the patient, the immuno-oncology agent comprising a lyophilized fecal-derived microbiota and a capsule encapsulating the lyophilized fecal-derived microbiota; administering a cancer treatment to the patient.
Clause 18. The method of clause 17, wherein the primary condition includes one or more of melanoma, non-squamous cell lung cancer, squamous cell lung cancer, renal cell carcinoma, head and neck tumors, bladder cancer, Hodgkin lymphoma, non-Hodgkin lymphoma, gastric cancer, colorectal cancer, multiple myeloma, esophageal cancer, breast cancer, glioblastoma, mediastinal B-cell lymphoma, other hematologic malignancies, testicular cancer, pancreatic cancer, lymphoma, cervical cancer, ovarian cancer, basal cell carcinoma, neuroblastoma, leukemia, and sarcoma.
19. The method of any one of clauses 17-18, wherein administering a cancer treatment to the patient occurs after administering an immuno-oncology agent to a patient with a cancer to enhance the immune system of the patient.
Clause 20. The method of any one of clauses 17-19, wherein administering an immuno-oncology agent to a patient with a cancer to enhance the immune system of the patient enhances the efficacy of the cancer treatment.
Clause 21. A method for treating breast cancer, the method comprising: administering an MRT composition to a patient with breast cancer prior to a primary breast cancer treatment; and treating the patient with the primary breast cancer treatment.
Clause 22. The method of clause 21, wherein administering an MRT composition to a patient with breast cancer includes administering one or more oral capsules to the patient.
Clause 23. The method of clause 22, wherein administering one or more oral capsules to the patient includes administering four capsules or more per day to the patient.
Clause 24. The method of clause 22, wherein administering one or more oral capsules to the patient includes administering up to four capsules per day to the patient.
Clause 25. The method of clause 22, wherein administering one or more oral capsules to the patient includes administering four capsules or more per day to the patient for two weeks.
Clause 26. The method of clause 22, wherein administering one or more orals capsules to the patient includes administering four capsules or more per day to the patient for two or more weeks.
Clause 27. The method of clause 22, wherein administering one or more orals capsules to the patient includes administering four capsules or more per day to the patient for four weeks.
Clause 27. The method of any one of clauses 22-27, wherein each oral capsule comprises 1 × 10⁷ to 1 × 10¹⁰ colony forming units.
Clause 28. The method of any one of clauses 22-27, wherein each oral capsule comprises 1 × 10⁷ to 1 × 10¹⁰ colony forming units of viable bacteria.
Clause 29. The method of any one of clauses 21-28, wherein treating the patient with the primary breast cancer treatment includes surgery.

## Claims

1. An immuno-oncology agent and a cancer treatment for use in enhancing the immune system of a patient with a cancer, wherein the immuno-oncology agent includes a microbiota restoration therapy (MRT) composition.

2. The immuno-oncology agent and cancer treatment for use of claim 1, wherein the MRT composition includes one or more oral capsules or an enema.

3. The immuno-oncology agent and cancer treatment for use of claim 1, wherein the cancer treatment includes immune-checkpoint therapy.

4. The immuno-oncology agent and cancer treatment for use of claim 3, wherein the immune-checkpoint therapy is an immune checkpoint inhibitor, optionally selected from PD-1 inhibitors, PD-L1 inhibitors, and CTLA-4 agents.

5. The immuno-oncology agent and cancer treatment for use of claim 1, wherein the cancer treatment includes an immune-adoptive T-cell therapy.

6. The immuno-oncology agent and cancer treatment for use of claim 1, wherein the cancer treatment includes T cell-activating bispecific antibodies.

7. The immuno-oncology agent and cancer treatment for use of claim 1, wherein the cancer treatment includes unmethylated CG dinucleotides that are to be intratumorally injected.

8. The immuno-oncology agent and cancer treatment for use of claim 1, wherein the cancer treatment includes a cell therapy, optionally a CAR-T therapy.

9. The immuno-oncology agent and cancer treatment for use of any one of claims 1 to 8, wherein the MRT composition enhances the immune system by one or more of: increasing T-cell activation, inducing interferon-gamma-producing CD8 T-cells, increasing the infiltration of T-cells into tumors, increasing infiltration of lymphocytes into tumors, increasing infiltration of interferon gamma-producing CD8 T-cells, decreasing CD4+ regulatory T-cells in tumors, and inducing other cells selected from CD3, PD1, and FoxP3 producing T-cells, or combinations thereof

10. The immuno-oncology agent and cancer treatment for use of claim 1, wherein the cancer is melanoma, non-squamous cell lung cancer, squamous cell lung cancer, renal cell carcinoma, head and neck tumors, bladder cancer, Hodgkin lymphoma, non-Hodgkin lymphoma, gastric cancer, colorectal cancer, multiple myeloma, esophageal cancer, breast cancer, glioblastoma, mediastinal B-cell lymphoma, other hematologic malignancies, testicular cancer, pancreatic cancer, lymphoma, cervical cancer, ovarian cancer, basal cell carcinoma, neuroblastoma, leukemia, and sarcoma.

11. The immuno-oncology agent and cancer treatment for use of claim 1, wherein the cancer treatment is a breast cancer treatment for use in treating breast cancer in a patient with breast cancer, and wherein the MRT composition is to be administered prior to the primary breast cancer treatment.

12. The immuno-oncology agent and cancer treatment for use of any one of claims 1-11, wherein each oral capsule comprises 1 × 10⁷ to 1 × 10¹⁰ colony forming units.

13. The immuno-oncology agent and cancer treatment for use of any one of claims 11-12, wherein the primary breast cancer treatment includes surgery.
